# EUROPEAN PATENT APPLICATION

(11) **EP 4 552 840 A1**
(43) Date of publication of application: **14.05.2025**
(21) Application number: 24207044.9
(22) Date of filing: 16.10.2024
(51) Int. Cl.: B32B 5/02, B32B 5/06, B32B 5/08, B32B 5/26, A23G 4/20, A24B 13/00, A61K 9/24, D04H 1/425, D04H 1/46, D04H 1/498, D04H 1/4258, D04H 1/541, D04H 1/542

(54) **NONWOVEN PRODUCT FOR ORAL DELIVERY OF A SUBSTANCE, AND METHOD OF MANUFACTURING THE SAME**

(30) Priority: 10.11.2023 GB 202317246
(71) Applicant: Nonwovenn Ltd, Bridgwater, Somerset TA6 4NZ (GB); Contraf-Nicotex-Tobacco GmbH, 74072 Heilbronn (DE)
(72) Inventor: GENTILCORE, Giovanni, Bridgwater, TA6 4NZ (GB); HILL, Dave, Bridgwater, TA6 4NZ (GB); ELLERICHMANN, Thomas, D-74072 Heilbronn (DE); BELZ, Markus, D-74072 Heilbronn (DE); KÖNIG, Thorsten, D-74072 Heilbronn (DE)
(74) Representative: Mewburn Ellis LLP

(57) **Abstract**

A product for oral delivery of a substance in which the substance is retained with a layered structure of nonwoven materials that provides structural integrity capable of withstanding a chewing action, together with a desirable mouth feel and appealing product form factor. The substance may be a gel material that is held within a plurality of layers of nonwoven fabric that are secured together as a consolidated web by physical consolidation technique such as hydroentanglement, stitching, embossing, needle felting/punching.

## Description

### Field of the Invention

The present invention relates to a product fabricated from nonwoven materials, where the product provides a means for oral delivery of a substance, e.g. an active substance such a pharmaceutical or other chemical. The present invention also relates to a method of manufacturing such a product. The product may be provided in a dosage form such as a lozenge, tablet, pastille, tearable strip or the like. The nonwoven materials of the product may be configured both to carry the substance and to withstand oral use, e.g. be strong enough to be chewable.

### Background

It is known to use such nonwoven fabrics to manufacture products for oral delivery of substances. For example, nonwoven fabrics are used to make pouches for containing an individual portion of a product, such as smokeless tobacco (also known as "snus"), coffee, tea, etc., from which flavour is to be delivered. Examples of oral pouched products formed from a nonwoven fabric can be found in US 2014/0026912 A1 and US 2012/0103353 A1.

Nonwoven fabrics are typically produced using one of three processes: dry-laid, wet-laid, or spun melt. Each process entangles fibres or filaments into a web in a manner that does not require weaving or knitting.

A dry-laid process typically comprises forming a loose web of staple fibres. Such webs are subsequently bonded together to create the fabric. The web may be formed by an air laying process, whereby the fibres are randomly orientated in the web. Alternatively, forming the web may include carding the fibres, which aligns their orientation. The air laid or carded web can be bonded using mechanical (e.g. hydroentanglement, stitching, embossing or needle felting), thermal (e.g. where the web includes thermoplastic fibres) or chemical techniques (e.g. using an adhesive binder), or a combination thereof.

A wet-laid process typically comprises forming a slurry of fibres in water or other suitable liquid, which is deposited on a screen or mesh and then dried to form the web.

A spun melt process typically forms a web from continuous filaments spun directly from liquid (i.e. melted) plastic materials.

Typically, the nonwoven fabrics used to produce pouched products are water-permeable, in order to permit substances (e.g. flavour) from the contents of the pouch to flow out.

Nonwoven fabric may be used to manufacture chewable pouches. For example, US 2018/0153211 A1 discloses a nonwoven fabric for manufacturing a pouched product, in which the pouch includes an elastic mesh of thermoplastic polyamide that ensures the pouch can endure repeated deformations caused by chewing. The elastic mesh in this example has a high percentage of open area (i.e. high porosity) to enable a rapid release rate of flavour from the pouch.

US 2013/0149254 A1 discloses a perforated chewable pouch made of a food grade material selected from silicone, latex, rubber, or plastic. The pouch encloses a product that can be in a gel, semi-liquid, and/or liquid form. A user chews, sucks, and/or manipulates the pouch to cause the enclosed flavour product to leach out of the perforations into the user's mouth.

US 2014/0261480 A1 discloses a pouch formed from a fabric comprising melt-blown polymer fibres having a hydrophilic surface coating. The melt-blown material can be an elastomer (e.g. polymeric polyurethane) so that the pouch can tolerate being chewed.

Other form factors for nonwoven oral products have also been conceived. For example, WO2022/112085 discloses a chewable product for oral delivery of a substance, wherein the chewable product is an essentially homogenous portion of nonwoven fabric impregnated with the substance.

The present invention has been devised in light of the above considerations.

### Summary of the Invention

At its most general, the invention provides a product for oral delivery of a substance in which the substance is retained with a layered structure of nonwoven materials that provides structural integrity capable of withstanding a chewing action, together with a desirable mouth feel and appealing product form factor. In one embodiment, the substance is a gel material that is held within a plurality of layers of nonwoven fabric that are secured together as a consolidated web by physical consolidation technique such as hydroentanglement, stitching, embossing, needle felting/punching.

The gel material may provide a convenient carrier medium for an active agent, such as a pharmaceutical material, smokeless tobacco, nicotine salts, free nicotine base, cannabidiol, coffee, tea, flavouring or the like. For example, the active agent may consist or comprise any one or more of: amino acids, in particular taurine, lysine, tryptophane, theanine, tyrosine, or 5-HTP; alkaloids, in particular nicotine, anatabine, caffeine, theobromine or theophylline; natural or synthetic cannabinoids, in particular cannabinol, cannabidivarin, cannabichromen, cannabidiol, cannabigerol, tetrahydrocannabivarin, Delta-8-tetrahydrocannabinol, Delta-9-tetrahydrocannabinol or Delta-x-tetrahydro cannabidiol; hormones, in particular melatonin; vitamins or minerals. The layered structure of nonwoven materials may provide a robust framework for holding the gel material to permit release of the active agent in an aqueous environment, e.g. within a user's mouth.

According to a first aspect of the invention, there is provided a product for oral delivery of a substance, the product comprising: a layered structure comprising a plurality of nonwoven fabric layers that include an intermediate carrier layer and a pair of support layers; and a substance held by the intermediate carrier layer, wherein the intermediate carrier layer and substance are sandwiched between the pair of support layers, and wherein the layered structure is mechanically consolidated to secure together the plurality of nonwoven fabric layers. In use the product is for placing in a user's mouth to be sucked and/or chewed to release the substance (or a component, e.g. active agent thereof) from the nonwoven fabric layers.

The mechanical consolidation of the layered structure may be result in fibres within each of the constituent layers becoming entangled to hold the layers together. In one example, the layered structure is mechanically consolidated by needle punching.

The substance may be or may include a component for consumption by the user. For example, the substance (or active agent thereof) may comprise any of smokeless tobacco, nicotine salts, cannabidiol, coffee, tea, flavouring or the like. The substance may comprise one or more media configured to remove impurities, e.g. active charcoal, or zeolites. The substance may comprise a pharmaceutical material such as aspirin, paracetamol, ibuprofen or another API.

The substance may comprise a carrier medium within which the component (e.g. active agent) for consumption is contained. For example, the substance may comprise a carrier medium having the active agent distributed therein. The carrier medium may be a gel, e.g. an aqueous gel (i.e., hydrogel) formed by mixing water with a suitable gelling agent, such as agar-agar, alginates, carrageenan, cellulose derivates, in particular carboxy methylcellulose and hydroxypropyl methylcellulose, polyvinyl pyrrolidone, polyvinyl acetate, and mixtures of at least two of the afore-said hydrogel forming components.

The intermediate carrier layer may have a different colour from the pair of support layers. In use, the intermediate carrier layer may be visible around the edge of the product, and providing a different colour for the intermediate carrier layer can therefore enable the intermediate carrier layer to be used as an identifier for the product, e.g. to indicate the nature of the substance contained therein, e.g. its flavour or the identity of the active agent.

The intermediate carrier layer may be a darker colour than the pair of support layers. For example, the pair of support layers may be light-coloured, e.g. white or light brown, whereas the intermediate carrier layer may be dark-coloured, e.g. black or blue. Having a darker colour of the intermediate carrier layer may be advantageous in masking discoloration of the substance in use (e.g. caused by oxidation of the active agent).

The intermediate carrier layer may comprise a single layer on which this substance is disposed. Alternatively, the intermediate carrier layer may comprise a base substrate layer and an upper substate layer, wherein the substance is disposed between the base substrate layer and the upper substrate layer. The intermediate carrier layer thus resembles a sandwich in which the substance (e.g. gel material) is held within two surrounding layers. The base substrate layer and the upper substrate layer are preferably made from the same nonwoven material. This may assist in providing a uniform appearance for the product. The base substrate layer and the upper substrate layer may act to confine the substance, i.e., prevent the active agent from contacted outer layers of the layered structure.

The base substrate layer and/or the upper substate layer may comprise a nonwoven fabric in which a matrix of staple fibres are bonded together by a binder. For example, the base substrate layer and/or the upper substate layer may comprise a conventional dry-laid chembond nonwoven material, e.g. formed from viscose fibres that are chemically (e.g. adhesively) bonded together with a suitable binder.

To provide a colour to the intermediate carrier layer, the binder may include a pigment or dye.

As the product is intended for oral use, the binder and any colouring agent (e.g. pigment dispersion or dye) mixed therewith is preferably a food-grade substance.

Each of the pair of support layers may comprise a nonwoven material having a weight equal to or greater than 30 gsm, preferably equal to or greater than 40 gsm, more preferably equal to or greater than 50 gsm. Using a nonwoven material with this weight may enable the pair of support layers to provide protection for the intermediate carrier layer and bulk for the product itself.

The pair of support layers may comprise food-grade staple fibres (e.g. viscose or the like) that are entangled by needle punching. The pair of support layers may thus consist of entangled nonwoven fibres without a separate binder. This ensures that the product has a satisfactory appearance and mouthfeel.

Each of the pair of support layers may comprise an inward support layer disposed next to the intermediate carrier layer and an outward support layer disposed over a surface of the inward support layer opposite the intermediate carrier layer. The inward support layer may be of a different nonwoven material from the outward support layer. For example, the outward support layer may comprise a mixture of staple fibres (e.g. viscose) and thermoplastic fibres (e.g. polypropylene, polyethylene, polyester, ethylene vinyl acetate, polyurethane, polylactic acid and polyamides). The advantage of this material is that it provides a soft mouthfeel. The inward support layer may consist solely of needled staple fibres.

The product may be configured as an individual portion, e.g. in the form of a tablet, lozenge, pastille or the like. The individual portion may have formed with any suitable shape. In one example, the layered structure is in the form of a substantially cuboidal tablet having a length of 20-40 mm, a width of 10-30 mm, and a thickness of 1-5 mm. The intermediate carrier layer may be visible around the edge of this structure, e.g. as a strip of colour. In one example, the product has a length of 30 mm and width of 12 mm.

Alternatively, the product may be configured as a multi-portion article, from which a user can separate pieces to be consumed. For example, the layered structure may be in the form of a strip having a length equal to or greater than 100 mm, a width of 10-30 mm, and a thickness of 1-5 mm. The strip may be torn by the user, e.g. at lines of weakness, to obtain an individual portion. The user may be able to select the size of the portion, e.g. according to the length of the section of the strip that is torn off.

In another aspect, there is provided a method of manufacturing a product for oral delivery of a substance, the method comprising: applying a substance to a base substrate layer of nonwoven fabric to form an intermediate carrier layer that holds the substance; laying support layers of nonwoven fabric on either side of the intermediate carrier layer to form a layered web; and mechanically consolidating the layered web to secure together the base substate layer and the support layers in a consolidated layered structure.

The method may be used to make a product as discussed above, and therefore features of the first aspect are equally applicable to this aspect.

The method uses physical entanglement of nonwoven fibres (e.g. obtained by needle punching or other mechanical consolidation technique) to provide the product with the structural strength required to withstand oral use. This is advantageous both for the appearance of the final product and to minimize the need for additional chemicals in the product, e.g. because a binder is not required in the support layers.

The step of mechanically consolidating the layered web may comprise passing the layered web through a needle loom that performs needle punching to entangle fibres in the layered web to form the consolidated layered structure.

The method may include laying an upper substrate layer of nonwoven fabric over the base substate layer to cover the substance, wherein the upper substrate layer and base substrate layer form the intermediate carrier layer for holding the substance.

The method may include one of more steps to further process the consolidated layered structure, e.g. for storage, transportation, or packaging. For example, the method may include cutting the consolidated layered structure into a plurality of portions. The portions may be the individual tablet-like portions or the multi-portion strips discussed above. The method may include packaging the plurality of portions, e.g. individually wrapping each portion or placing portions in a suitable container.

The step of applying the substance may comprise: preparing a gel material, and depositing the gel material on the base substrate layer. The gel material and/or the base substrate layer may be heated during this step. For example, the gel material may be prepared by mixing gel precursor components at a mixing temperature, e.g. around 70°C. It may be advantageous to apply the gel mixture at an elevated temperature to assist in uniform spreading on the base substrate layer and/or to promote ingress or migration of the gel material into the surrounding nonwoven fabric while the gel mix is still in a flowable state. For example, the gel material may flow more readily at an elevated temperature. The base substrate layer and/or the gel mixture may thus be heated to an application temperature when the gel material is deposited. The application temperature may be equal to or greater than 30°C to ensure suitable flowability. The application temperature may be equal to or less than 45°C, and preferably equal to or less than 40°C to minimise evaporative loss of volatile components of the gel mix (e.g. a flavour component or the like). The application temperature may preferably be any temperature in the range 30-40°C. The substrate to which the gel mixture is applied may be heated to the application temperature independently of any heating applied to the gel material.

The heating may be localised, e.g. at the point of depositing the gel material. For example, a conventional hot melt applicator may be adapted to pour, roll, spray or extrude the gel material on to the heated base substrate layer. Alternatively, the heating may be beneficially applied to other steps of the method. For example, any one of more of the steps of laying the upper substrate layer, laying the support layers, and passing the layered web through a needle loom may be performed at the application temperature. This may assist in integrating the gel material within the layered web.

The step of preparing the gel material may comprise mixing a dry phase precursor and a wet phase precursor with water at the application temperature. The dry phase precursor may include a gelling agent. The wet phase precursor may comprise an active agent. This arrangement allows the gel material to be formed at the point of application.

The substance may include one or more flavour components in addition to the active agent. Alternatively, one of more flavour components may be applied to the product separately from the active agent, e.g. as an additional layer or as a coating on the final product. For example, the active agent may be applied using the gel material discussed above, whereas flavour may be applied as a separate layer or coating.

The invention includes the combination of the aspects and preferred features described except where such a combination is clearly impermissible or expressly avoided.

### Summary of the Figures

Embodiments and experiments illustrating the principles of the invention will now be discussed with reference to the accompanying figures in which:
Fig. 1 is a schematic drawing of a nonwoven product that is an embodiment of the invention;
Fig. 2A is a schematic cross-section through a first example of the nonwoven product of Fig. 1;
Fig. 2B is a schematic cross-section through a first example of the nonwoven product of Fig. 1;
Fig. 3 is a schematic drawing of an apparatus for manufacture of a nonwoven product that is an embodiment of the invention;
Fig. 4 is a flowchart of a method for manufacturing a nonwoven product that is an embodiment of the invention;
Fig. 5 is a schematic drawing of an alternative applicator module that can be used in the apparatus of Fig. 3; and
Fig. 6 is a schematic drawing of another nonwoven product that is an embodiment of the invention.

### Detailed Description of the Invention

Aspects and embodiments of the present invention will now be discussed with reference to the accompanying figures. Further aspects and embodiments will be apparent to those skilled in the art. All documents mentioned in this text are incorporated herein by reference.

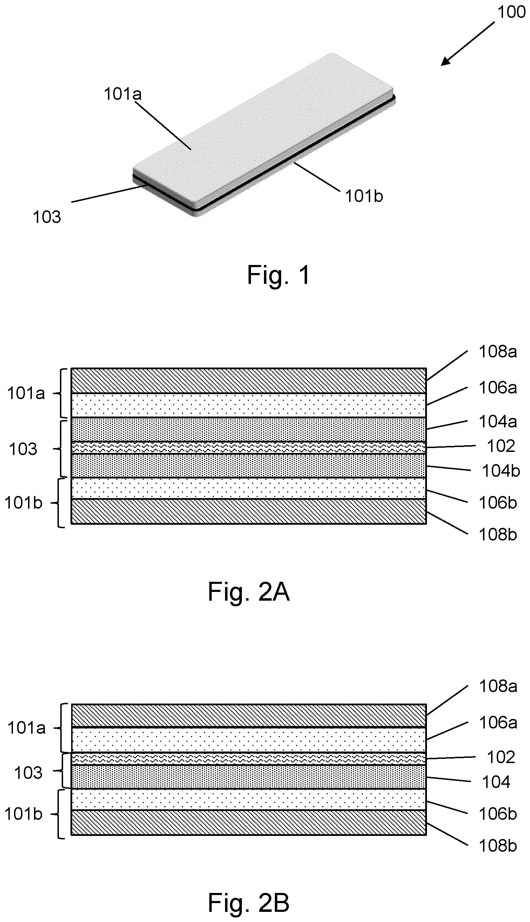

Fig. 1 shows a schematic perspective view of a nonwoven product 100 that is an embodiment of the invention. The nonwoven product 100 is a portion of a layered structure that comprises an upper support layer 101a, a lower support layer 101b and an intermediate carrier layer 103.

The upper and lower support layers 101a, 101b are formed from nonwoven material that is selected to be robust enough for oral use, i.e. to withstand chewing when in a wet state. It is desirable for the nonwoven material of these support layers to have a tensile strength that can withstand the forces associated with chewing. For example, the nonwoven material may have a tensile strength above a predetermined threshold when in a wet state, e.g. saturated with water or the like. The tensile strength when in a wet state may be referred to herein as a "wet strength" of the nonwoven fabric substrate. The fibres in the nonwoven material may be generally aligned in a first direction (referred to herein as the machine direction) that is perpendicular to a second direction (referred to herein as the cross direction). A wet strength of the nonwoven material may be defined in each of the machine direction and the cross direction. In one example, the nonwoven material preferably has a wet strength that is equal to or greater than 5 N/cm in the cross direction and/or 30 N/cm in the machine direction. The tensile strength of the nonwoven material when in a dry state may be equal to or greater than 15 N/cm in the cross direction and equal to or greater than 15 N/cm in the machine direction.

The tensile strength of a nonwoven material (both in a dry state and in a wet state) may be measured using the grab strength test method published as NWSP 110.1 by EDANA.

The upper and lower support layers 101a, 101b may be made from the same material so that the intermediate carrier layer 103 is surrounded by the same type of support on both sides. The upper and lower support layers 101a, 101b provide physical protection for the intermediate layer. As discussed below in more detail, the upper and lower support layers 101a, 101b may be formed from nonwoven materials that are approved for use in food packaging (e.g. ISEGA certified) and which are capable of providing the desired physical dimensions and mouthfeel for the product.

The intermediate carrier layer 103 comprises a layer of nonwoven material that acts as a substrate to carry or otherwise support a substance (e.g. an active substance) that is released in use, i.e. when the product is in the user's mouth. The substance may be supported (e.g. sandwiched) between a pair of nonwoven layers. As shown in Fig. 1, the intermediate carrier layer 103 may have a different (e.g. contrasting) colour from the upper and lower support layers 101a, 101b. In one example, the intermediate carrier layer 103 has a darker colour than the upper and lower support layers 101a, 101b. For example, the upper and lower support layers 101a, 101b may be a light colour, e.g. substantially white, and the intermediate carrier layer 103 may have a contrasting colour, e.g. red, blue, green, black, etc. The colour may be provided as a pigment or ink in the nonwoven material of the carrier layer. For example, the carrier layer may include a layer of dry-laid nonwoven material in which staple fibres are chemically bonded together using a binder. The binder may be used as the means to colour the material, e.g. by including a pigment dispersion or the like, or the staple fibres themselves may be coloured..

Colouring the intermediate carrier layer 103 may provide two advantages. Firstly, it may mask and discolouration of the nonwoven substrate that carries the substance. For example, where the substance includes nicotine, there will be natural discolouration over time due to exposure of the nicotine to oxygen in the air. This discolouration will not be visible if the nonwoven substrate that carries the substance is already dark. Secondly, the colour of the intermediate carrier layer may act as an identifier, thereby allowing different types of products to be visually distinguished. For example, the colour may be an identifier for the substance, e.g. to allow nicotine-containing products to be distinguished from non-nicotine products or to allow different flavours (e.g. mint, lemon, etc.) to be distinguished. Additionally or alternatively, the colour may be used as an identifier for the type of nonwoven material used in the support layers, e.g. as an indicator of different mouthfeel.

As shown in Fig. 1, the nonwoven product 100 takes the form of a substantially cuboidal tablet or lozenge, but it may be understood that other shapes can be used, e.g. cylindrical pill, disc-shaped tablet, etc.

The lozenge may be sized to fit comfortably in a user's mouth to be chewed or sucked. It made be sized to fit under a user's upper or lower lip, e.g. between the lip and teeth. For example, the product 100 may have a length greater than 20 mm, e.g. in the range 20-40 mm, preferably 30 mm. The product 100 may have a width greater than 10 mm, e.g. in the range 10-30 mm, preferably 12 mm. The product 100 may have a thickness greater than 1 mm, e.g. in the range 1-5mm, preferably 2-3 mm. The length and width of the product may be selected by cutting a larger sheet of the layered structure into individual portions.

The thickness of the product may be achieved through the steps of the fabrication process set out below.

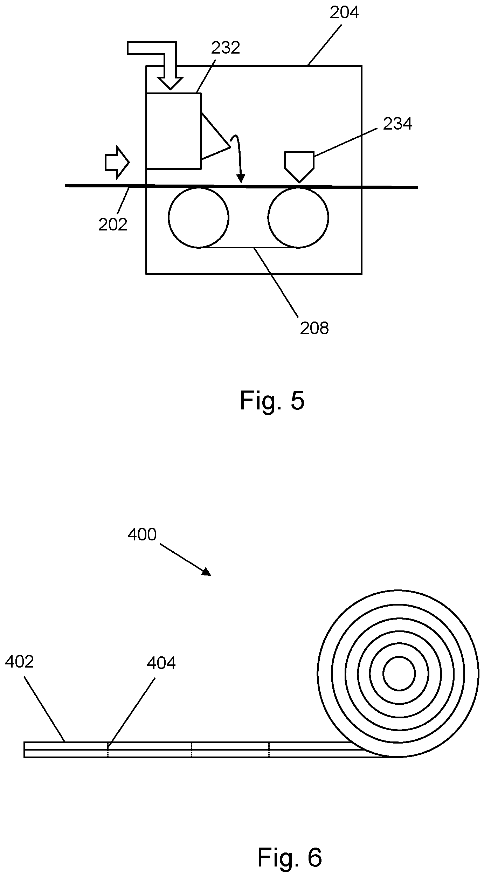

In another example, the nonwoven product 100 may be provided as a longer strip of material, e.g. having the same width and thickness discussed above, but a much longer length. The strip can be provided in a rolled form (see Fig. 6). In use a portion (e.g. having a length similar to that discussed above) can be torn off the strip. The total length of the strip may be greater than 100 mm, e.g. in the range 100-500mm, preferably 300 mm. The strip may have lateral perforations or other lines of weakness provided at regular intervals along its length to demarcate individual portions.

Figs. 2A and 2B are schematic cross-sectional views through two examples of the nonwoven product 100 of Fig. 1. In these examples, each of the upper and lower support layers 101a, 101b comprises two layers. The invention need not be limited to this example. The upper and lower support layers 101a, 101b may each consist of a single layer, or may each comprise three or more layers. In the examples shown in Figs. 2A and 2B, each of the upper and lower support layers 101a, 101b has an inward layer 106a, 106b and an outward layer 108a, 108b. Each inward layer 106a, 106b contacts a respective surface of the intermediate support layer 103. Each outward layer 108a, 108b has a surface that is exposed on the outside of the product. A specific example of the composition of these layers is set out in Table 1 below.

The nonwoven materials used in the upper and lower support layers 101a, 101b are preferably dry-laid needled fabrics. Using needled fabrics can assist in giving the layered structure the bulk (i.e. thickness) that is desired in the product. The needled fabrics may have a weight greater than 50 gsm, e.g. in the range 50-100 gsm, preferably 80-90 gsm. This weight gives the layers structural integrity that is capable of withstanding the physical forces encountered when a user chews the product.

The needled fabrics may comprise staple fibres of a material that complies with food contact regulations, i.e. is approved for use in food packaging or the like. The staple fibres may be natural fibres such as cotton or bamboo, or regenerated cellulosic fibres such as viscose and/or lyocell. For example, the needled fabrics may by formed predominantly or solely from viscose fibres. The viscose fibres used in the upper and lower support layers may have a fibre density greater than 2.5 dtex, e.g. in the range 2.5-5 dtex, preferably 4-4.5 dtex. Is some examples, upper and lower support layers 101a, 101b may include one or more layers adjacent to the intermediate carrier layer 103 formed from fibres having a fibre density less than 2.5 dtex. This may improve migration of the substance into the support layer, which in turn assist in transfer to the user's mouth in use.

The viscose fibres used in the upper and lower support layers may have an average fibre length in the range 40-80 mm, preferably 60 mm. Fibres of this length may facilitate both the creation and integrity of the 3D structure.

The outward layers 108a, 108b may also include thermoplastic fibres blended with the staple fibres. For example the fibre composition of the outward layers 108a, 108b may comprise 10-50 wt% thermoplastic fibres, preferably 20-30 wt%. Such a blend of fibres may provide a softer mouthfeel and is therefore beneficial to have on the outside of the product. The thermoplastic fibres may consist of any one or more of: polypropylene, polyethylene, polyester, ethylene vinyl acetate, polyurethane, polylactic acid and polyamides. These materials are preferred because they comply with food contact regulations and are hence suitable for products intended for intra-oral use. In one example, the thermoplastic fibres may comprise a polypropylene/polyethylene bicomponent fibres. An advantage of such fibres is that the lower melting point of the sheath material enables seals to be made at lower temperatures whilst the core material maintains structure and integrity.

The intermediate carrier layer 103 in the example shown in Fig. 2A comprises a pair of substrate layers 104a, 104b which surround and support a central layer 102 that comprises or consists of the substance that is released in use. It may be useful to provide the substance in a central layer 102 having substrate layers 104a, 104b on each side thereof to provide a uniform appearance and protection for the substance, as shown in Fig. 2A. The substrate layers 104a, 104b may have identical compositions. For example, each substrate layer 104a, 104b may be a dry-laid carded nonwoven fabric of viscose fibres having a fibre density of 1.7 dtex that are chemically (e.g. adhesively) bonded together with a suitable binder. The binder is preferably a food-grade substance, i.e. a substance that complies with regulations on food contact. Such substances include vinyl acrylic copolymer, vinyl acetate ethylene copolymer and a vinyl acetate copolymer. In one example, the binder is a mix of a vinyl acrylic copolymer binder and an acrylic copolymer binder. The binder may also preferably include a biodegradable material such as a polylactic acid (PLA) emulsion or a poly(butylene succinate) (PBS) emulsion.

In another example, the intermediate carrier layer 103 may consist of a single substrate layer 104 that supports the substance to be released, as shown in Fig. 2B. This single substrate layer 104 may have the same composition as the lower substrate layer 104b listed in Table 1. The single substrate layer 104 or the substrate layers 104a, 104b may be coloured as discussed above.

**Table 1: Nonwoven layer properties**

| *Layer* | *Type* | *Fibre* | *Weight* |
|---|---|---|---|
| Outward layer 108a | Dry-laid needled | 75% viscose (4.2 dtex, 60 mm) | 85 gsm |
| | | 25% PP/PE bicomponent (2.2 dtex, 40 mm) | |
| Inward layer 106a | Dry-laid needled | 100% viscose (3.1 dtex, 40 mm) | 90 gsm |
| Upper substate layer 104a | Dry-laid chembond | 70% viscose (1.7 dtex, 40 mm) | 32 gsm |
| | | 30% co-polyester bicomponent (2.5 dtex, 51 mm) | |
| Lower substate layer 104b | Dry-laid chembond | 70% viscose (1.7 dtex, 40 mm) | 32 gsm |
| | | 30% co-polyester bicomponent (2.5 dtex, 51 mm) | |
| Inward layer 106b | Dry-laid needled | 100% viscose (3.1 dtex, 40 mm) | 90 gsm |
| Outward layer 108b | Dry-laid needled | 75% viscose (4.2 dtex, 60 mm) | 85 gsm |
| | | 25% PP/PE bicomponent (2.2 dtex, 40 mm) | |

Although depicted as a separate layer 102 in Figs. 2A and 2B, it is to be understood that in practice the substance contained within the product can seep into the surrounding layers so that it is at least partially held within the matrix of nonwoven fibres that make up those surrounding layers. For example, in the arrangements shown in Figs. 2A and 2B, the substance may exist in both the central layer and within the substrate layers 104, 104a, 104b. In some examples the substance may predominantly subsist in the substrate layer(s), i.e. the thickness of the central layer may be negligible in the final product.

In this example, the central layer 102 comprises a carrier medium that releasably supports the substance to be released, e.g. to permit an active agent to be released in use (i.e. when the product is chewed or sucked by in the user's mouth). The substance (or active agent thereof) may comprise any of smokeless tobacco, nicotine salts, cannabidiol, coffee, tea, flavouring or the like. The substance may comprise one or more media configured to remove impurities, e.g. active charcoal or zeolites. The substance may comprise a pharmaceutical material such as aspirin, paracetamol and ibuprofen.

The carrier medium is preferably a food-grade material that is both suitable for application to and integration with nonwoven fabrics and which is capable of retaining the substance (or active agent thereof) for extended periods before the product is used. For example, the carrier medium may be a gel layer that is formed between the substrate layers 104a, 104b during manufacture of the product. The gel layer may be formed using a suitable food grade gelling agent to which the active agent (e.g. nicotine) and other flavourings (carried within propylene glycol or other suitable carrier) are added.

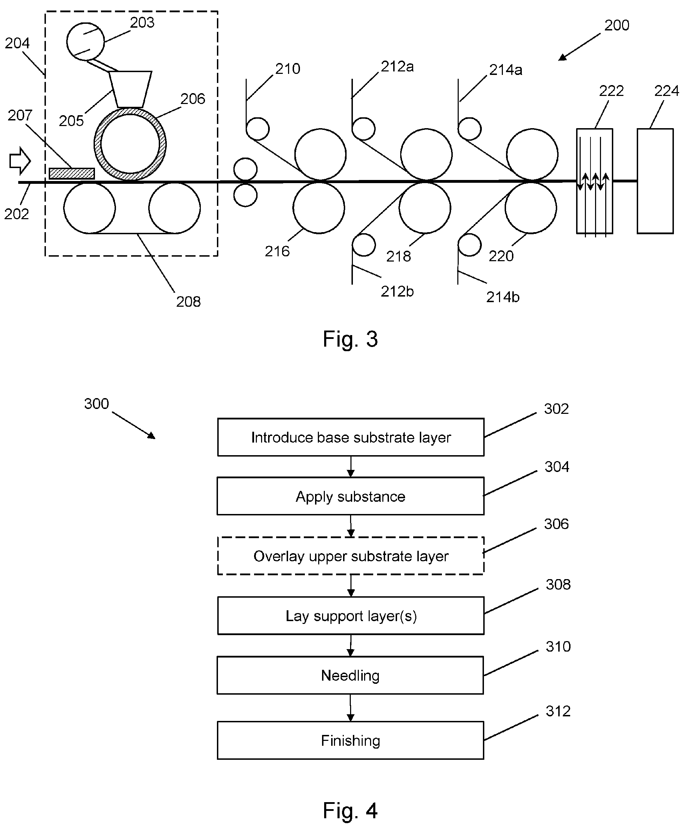

Fig. 3 is a schematic drawing of an apparatus 200 for manufacture of a nonwoven product that is an embodiment of the invention. In general, the apparatus 200 is arranged to deposit a substance on a substrate layer of nonwoven fabric, which is then combined (i.e. overlaid or underlaid) with additional layers of nonwoven fabric. The multi-layer structure is then physically consolidated, e.g. by needling, after which it can be sent for final processing into the final product form.

In the apparatus 200 shown in Fig. 3, a first conveyor 208 transports a base (lower) substrate layer 202 of nonwoven fabric into a gel application zone 204. The base substrate layer of nonwoven fabric 202 is a dry-laid carded chembond nonwoven fabric as discussed above. This nonwoven fabric may be manufactured in advance in a conventional manner, such as that described in WO 2021/219494, for example.

The gel application zone may resemble a so-called 'hot melt' coating apparatus, e.g. used for applying layers of adhesive film or the like. The gel application zone 204 comprises a heated applicator 206 that is configured to deposit a layer of gel material on to the substrate layer 202. The applicator 206 may be a heated roller as shown in Fig. 3, or alternatively may be a spray or extruder. A heater 207 is disposed adjacent the substrate layer 202 upstream of the applicator 206. The heater 207 is arranged to heat the substrate layer 202 so that it has a temperature substantially the same as that of the applied gel material. The desired temperature may be equal to or greater than 40°C, e.g. in the range 40-60°C.

The gel material is prepared in a mixer 203 by mixing a dry phase precursor (including the gelling agent) and a wet phase precursor (comprising the active agent, if used) with water at an elevated temperature, e.g. equal to or greater than 40°C, e.g. in the range 40-60°C. Table 2 provides an example of the materials that may be present in the precursors.

**Table 2: Example gel composition**

| | *Material* | *Amount (wt% of final gel)* |
|---|---|---|
| *Dry phase* | Sweeteners | 106 |
| | Salt | 10 |
| | Gelling agent | 1.2 |
| *Wet phase* | Humectant | 200 |
| | Active agent | 5.0 |
| | Flavourings | 2.5 |
| *Water* | | 59.7 |

Following mixing, the gel material is fed to the applicator 206 from a feed funnel 205. It is desirable for the mixing and application of the gel material to take place at a uniform temperature to ensure that the gel exhibits enough liquidity to ensure even coverage on the substrate layer. The speed with which the base substrate layer 202 passes the applicator 206 is used to control the thickness of the gel layer that is applied. In one example, the system is arranged to deposit the gel layer with a thickness of 2.5 mm. The thickness of this layer in the final product may be controlled by setting the parameters of subsequent steps, e.g. the needling and finishing steps discussed below.

After application of the gel layer, the base substrate layer 202 is conveyed out of the gel application zone 204 to a first set of rollers 216 at which an upper substrate layer 210 is laid over the gel layer to form a sandwich structure that corresponds to the intermediate carrier layer 103 discussed above. The upper substrate layer 210 may be a dry-laid carded chembond nonwoven fabric of the same type as the base substrate layer 202.

After application of the upper substrate layer 210, the sandwich structure is conveyed to a second set of rollers 218 at which a pair of inward support layers 212a, 212b are laid respectively on the upper and lower surfaces of the sandwich structure. The support layers 212a, 212b may correspond to the inward support layers 106a, 106b discussed above, i.e. they may be dry-laid needled fabrics that provide bulk to the product and protection to the sandwich structure. One example of needling parameters for preparation of the inward support layers may be as follows:

**Table 3: Needling parameters for inward supporting layer**

| | |
|---|---|
| Needle type & gauge | Groz Beckert 15×15×38, Regular Barb |
| Needle density | 18.5 needles/cm² |
| Stroke rate | 246 rpm |
| Penetration depth | 16.0 mm |
| Web input speed | 4.5 m/min |
| Web output speed | 655 m/min |

After application of the inward support layers 212a, 212b, the structure is conveyed to a third set of rollers 220 at which a pair of outward support layers 214a, 214b are laid respectively on the exposed surfaces of the inward support layers 212a, 212b. The outward support layers 214a, 214b may correspond to the outward support layers 108a, 108b discussed above, i.e. they may be dry-laid needled fabrics comprises a blend of viscose and thermoplastic fibres to provide bulk to the product and a soft mouthfeel. One example of needling parameters for preparation of the outward support layers may be as follows:

**Table 4: Needling parameters for outward supporting layer**

| | |
|---|---|
| Needle type & gauge | Groz Beckert 15×15×38, Regular Barb |
| Needle density | 47.5 needles/cm² |
| Stroke rate | 716 rpm |
| Penetration depth | 14.3 mm |
| Web input speed | 5.54 m/min |
| Web output speed | 716 m/min |

After application of the outward support layers 214a, 214b, the structure comprises a layered web that is then conveyed to a needling device 222 and subjected to needle punching. References herein to "needling" and "needled" fabrics are intended to refer to the process of needle punching, and fabrics obtained therefrom. Needle punching is a mechanical web consolidation technique in which an array of needles are reciprocally inserted and withdrawn into a web of fibres, usually in a direction perpendicular to a plane of the web. Needle punching may be a particularly useful technique for physical consolidation because it is capable of producing fabrics with high tensile strength in three dimensions, i.e. the machine direction, the cross direction, and a thickness or height direction (also referred to as "loft") of the fabric. Moreover, through appropriate selection of the needle punching parameters, the loft, strength and density of the nonwoven fabric substrate may be controlled in a manner that enables repeatable consistent manufacture of a nonwoven product. When applied to the multi-layer structure of the present embodiment, the needling acts to intermingle or entangle the fibres of the various layers in a way that interconnects the layers, thereby prevent delamination of the product. The needling process also acts to distribute some of the gel material from the gel layer into the surrounding nonwoven fabric layers, so that the substance is present both in the gel layer and within the fibre matrix of the nonwoven material.

Table 5 below compares tensile strength measurements (taken in the machine direction) for each individual nonwoven layer in the multi-layer structure shown in Fig. 2B with the tensile strength of the multi-layered structure following mechanical consolidation (by needling). The measurements demonstrate that the needling process provides a significant increase in the tensile strength of the final product. This increased strength can contribute to the suitability of the product for oral use.

**Table 5: Tensile strength test results**

| *Material* | *Tensile strength (N*/*50 mm)* |
|---|---|
| Needled nonwoven fabric substrate (Fig. 2B) | 290.2 |
| Outward supporting layer 108a, 108b | 9.3 |
| Inward supporting layer 106a, 106b | 5 |
| Single substrate layer 104 (without substance 102) | 23 |

Table 6 below shows the results of delamination testing performed on the multi-layer structure shown in Fig. 2B following needling. Delamination was tested at the interface between each pair of nonwoven layers, with the results below given in order of the interfaces descending through the product as shown in Fig. 2B. The results demonstrate strong and substantially uniform resistance to delamination, even at the interface between the inward supporting layer 106a and single substrate layer 104 where the substance is present.

**Table 6: Delamination testing**

| *Layer interface* | *Delamination load (N*/*50 mm)* |
|---|---|
| Outward supporting layer 108a - inward supporting layer 106a | 3.8 |
| Inward supporting layer 106a - single substrate layer 104 | 3.4 |
| Single substrate layer 104 - Inward supporting layer 106b | 4.2 |
| Inward supporting layer 106b - outward supporting layer 108b | 4.9 |

The needle punching parameters may comprise any one or more of (i) needle geometry, (ii) needle density (i.e. the spacing between needles in the array of needles, and (iii) speed of perforation.

Relevant features of the needle geometry may comprise the size of the needle, e.g. length and outer diameter. The length may be defined in terms of penetration distance into the web. In some examples, the penetration distance may be equal to or greater than 10 mm, e.g. in the range 10-17 mm. The outer diameter may be defined by a wire gauge. For example, each needle may have a wire gauge in the range 32-38, i.e. an outer diameter in the range 0.1-0.2 mm. The needle may also comprise surface features, e.g. a barb or the like, to facilitate fibre entanglement.

The array of needles (which may also be referred to as a needle loom) may comprises a line of needles spaced in the cross direction. Such an array of needles preferably has a density equal to or greater than 400 needles/m, e.g. in the range 400-6000 needles/m.

The needle density influences the density of perforations or "stitches" formed in the web. The other factors that affect the stitch density are the speed of the web through a needle punching area and the stroke rate of the needle array, which corresponds to the speed of perforation. The speed of the web may be controllable at both an input to and an output from the needle punching area. In one example, the input and output speeds may be equal. In another example, the output speed may be less than the input speed, to promote entanglement and increasing fibre consolidation in the machine direction. Both the input and output speeds may be in the range 2-7 m/min. The stroke rate of the needle array is preferably equal to or greater than 180 rpm, e.g. in the range 180-750 rpm.

The needle punching area may be defined by a stripper plate, which may be a planar element that lies over the web to hold the web in place while the needles repeatedly penetrate it. The stripper plate may have a length in the machine direction equal to or greater than 10 mm, e.g. in the range 10-20 mm.

Following the needling process, the resulting consolidated web is conveyed from the needling device 222 to a finishing apparatus 224. The finishing apparatus 224 may include a crush laminator module configured to press the web into a desired final thickness (e.g., in the range 1-5 mm) through selection of suitable temperature, pressure and gap parameters. The finishing apparatus 224 may be further configured either to prepare the needled web for storage or transport or to process the web into a final product form factor. If the web is to be stored or transported, the finishing apparatus 224 may be configured to wind one or more sheets of the web into a roll of fabric. Alternatively or additionally, the finishing apparatus 224 may include a cutting device that operates to divide the web into a plurality of product portions. The cutting device may operate on the web when it is in either a flat or rolled form. The product portions may be individual portions that resemble a lozenge, tablet, or pastille, for example. Alternatively, the product portions may be strips of material, e.g. obtained by slicing the web when in a rolled form. Any suitable sheet cutting machine may be used for this purpose. The finishing apparatus 224 may forming lines of weakness, e.g. perforated lines or the like, in the web. In this final product, these lines of weakness may designate individual portions, e.g. for a user to manually separate. The finishing apparatus may further include devices for applying other surface treatment, e.g. embossing, printing or the like, to provide the product with a desired final appearance. Alternatively or additionally, the finishing apparatus may apply further colours or substances (e.g. flavouring or other active substance) to the surface of the product, e.g. by spraying, dipping or the like.

Fig. 4 is a flowchart of a method 300 for manufacturing a nonwoven product that is an embodiment of the invention. The method 300 may use the apparatus discussed above in relation to Fig. 3.

The method 300 begins with a step 302 of introducing a base substrate layer of nonwoven fabric into a zone for applying a substance thereto. The base substrate layer of nonwoven fabric is a dry-laid carded chembond nonwoven fabric as discussed above. As discussed above, it may be desirable for the zone in which the substance is applied to be maintained at an elevated temperature so that during application the substance maintains a physical state (e.g. flowable) that facilitates even spreading across the base substrate layer.

The method continues with a step 304 of applying a substance to the base substrate layer. As discussed above, the substance may be a gel material that is deposited as a layer, e.g. by rolling, spraying, extruding or the like. However, the method need not be limited to applying a gel material. For example, the substance may be applying by impregnating the base substrate layer with a solution (e.g. aqueous solution) or slurry of the substance. In this example, the base substrate layer may be dipped, sprayed, or otherwise coated in order to apply the substance. The method may include a drying and/or nipping step after the application of the substance in order to remove any excess material.

The method continues with an optional step 306 of applying an upper substrate layer of nonwoven fabric to the base substrate layer. Preferably this step involves laying the upper substrate layer over a surface of the base substrate layer on which the substance is deposited, e.g. to cover the substance. Where the substance is applied as a gel material, this step 306 may result in a layered structure that resembles a sandwich.

The method continues with a step 308 of laying support layers of nonwoven fabric on either side of the layered structure. As discussed above, the support layers may comprise a pair of inward support layers 212a, 212b and a pair of outward support layers 214a, 214b.

The method continues with a step 310 of needling the layered structure to consolidate the web may intermingling the materials of the various layers. For example, this step causes the fibres in the nonwoven layers to entangle with each other to give the web structural integrity. This step may also spread or otherwise cause migration of the substance between the upper and lower substrate layers (e.g. the gel material) into other layers of the layered structure, such that in the final product the substance is at least partially distributed within the matrix of the nonwoven layers.

The method continues with a step 312 of finishing the layered structure, e.g. by preparing a rolled web for storage or transportation, or by processing the web into the final product form factor, e.g. by performing any of rolling, cutting, and embossing, as discussed above. The final products may then be packaged, e.g. enclosed in a wrapper or other container.

The method may optionally include a step of drying the layered structure before or after needling. The drying step may be configured to control a moisture content for the final product. The moisture content in the final product may be in the range 0-60 wt%. Any conventional drying mechanism may be used. For example, the drying process may use any of hot air, heated roller, vacuum drying, etc.

As discussed above, the substance may be applied to the base substrate layer using an application module that resembles a so-called "hot melt" applicator. In that arrangement, the application module is arranged to heat the substrate layer to temperature corresponding to applied gel material. An alternative applicator arrangement is shown in Fig. 5. In this example, rather than heating the substrate layer directly, the gel application zone 204 instead comprises a housing that encloses a volume that is maintained at an elevated temperature (e.g. the desired temperature of application, which may be equal to or greater than 40°C). Within this heated volume, a layer of gel material can be deposited on the substrate layer 202 using a knife-over-roller technique, in which the gel material (following mixing of precursors as discussed above) is poured or otherwise deposited on to the substrate layer from a feed unit 232. The feed unit 232 may be a pouring vessel as shown in Fig. 5, or alternatively may be a roller, spray, or extruder. After the gel material is poured on to the substrate layer, the thickness of the gel layer is controlled by passing under a spreading blade 234. Excess gel material may be flow off the substrate layer and be collected, e.g. under the device.

The heated volume in the arrangement shown in Fig. 5 may be limited to the gel application zone. In this example, the subsequent steps of laying the support layers may occur in a relatively cooler environment, e.g. at ambient temperature. However, it is advantageous to maintain the entire fabrication environment at an elevated temperature so that the gel material retains a level of flowability that assists its integration into the rest of the fabric, e.g. by facilitating seepage into the substrate layers and migration into the support layers during needling. Alternatively, the elevated temperature may be maintained while the multi-layered structure is assembled, with the subsequent needling step occurring at a lower temperature, after the gel material has cooled.

Fig. 6 shows an alternative form factor for the product discussed above. In this example, the product 400 is formed as a strip 402 of material that can be rolled. The strip 402 is provided with a series of weakened regions 404 that permit sections of the strip to be torn off, e.g. for consumption. The weakened regions 404 may be provided at a regular, e.g. to demarcate individual portions. The weakened regions 404 may be perforated lines formed across the multi-layer structure. The total length of the strip 402 may be greater than 100 mm, e.g. in the range 100-500mm, preferably 300 mm. ***

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof.

While the invention has been described in conjunction with the exemplary embodiments described above, many equivalent modifications and variations will be apparent to those skilled in the art when given this disclosure. Accordingly, the exemplary embodiments of the invention set forth above are considered to be illustrative and not limiting. Various changes to the described embodiments may be made without departing from the spirit and scope of the invention.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

Throughout this specification, including the claims which follow, unless the context requires otherwise, the word "comprise" and "include", and variations such as "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means for example +/- 10%.

## Claims

1. A product for oral delivery of a substance, the product comprising:
a layered structure comprising a plurality of nonwoven fabric layers that include an intermediate carrier layer and a pair of support layers; and
a substance held by the intermediate carrier layer,
wherein the intermediate carrier layer and substance are sandwiched between the pair of support layers, and
wherein the layered structure is mechanically consolidated to secure together the plurality of nonwoven fabric layers.

2. The product of claim 1, wherein the layered structure is mechanically consolidated by needle punching.

3. The product of claim 1 or 2 wherein the intermediate carrier layer has a different colour from the pair of support layers.

4. The product of any preceding claim, wherein the intermediate carrier layer comprises a base substrate layer and an upper substate layer, and wherein the substance is disposed between the base substrate layer and the upper substrate layer.

5. The product of claim 4, wherein the base substrate layer and/or the upper substate layer comprise a nonwoven fabric in which a matrix of staple fibres are bonded together by a binder.

6. The product of claim 5, wherein the binder includes a pigment to impart a colour to the base substrate layer and/or the upper substate layer.

7. The product of any preceding claim, wherein each of the pair of support layers comprises a nonwoven material having a weight equal to or greater than 50 gsm.

8. The product of any preceding claim, wherein the pair of support layers comprise food-grade staple fibres that are entangled by needle punching and/or consist of entangled nonwoven fibres without a separate binder.

9. The product of any preceding claim, wherein each of the pair of support layers comprises an inward support layer disposed next to the intermediate carrier layer and an outward support layer disposed over a surface of the inward support layer opposite the intermediate carrier layer, and wherein the inward support layer is of a different nonwoven material from the outward support layer.

10. The product of claim 9, wherein the outward support layer comprises a mixture of staple fibres and thermoplastic fibres.

11. The product of any preceding claim, wherein the substance comprises a carrier medium and an active agent.

12. The product of any preceding claim, wherein the layered structure is in the form of a substantially cuboidal tablet having a length of 20-40 mm, a width of 10-30 mm, and a thickness of 1-5 mm.

13. The product of any one of claims 1 to 11, wherein the layered structure is in the form of a tearable strip having a length equal to or greater than 100 mm, a width of 10-30 mm, and a thickness of 1-5 mm.

14. A method of manufacturing a product for oral delivery of a substance, the method comprising:
applying a substance to a base substrate layer of nonwoven fabric to form an intermediate carrier layer that holds the substance;
laying support layers of nonwoven fabric on either side of the intermediate carrier layer to form a layered web; and
mechanically consolidating the layered web to secure together the base substate layer and the support layers in a consolidated layered structure.

15. The method of claim 14, wherein mechanically consolidating the layered web comprises passing the layered web through a needle loom that performs needle punching to entangle fibres in the layered web to form the consolidated layered structure.
